# EUROPÄISCHE PATENTSCHRIFT

(11) **EP 1 732 487 B1**
(45) Veröffentlichungstag und Bekanntmachung des Hinweises auf die Patenterteilung: **10.10.2007**
(21) Anmeldenummer: 05716249.7
(22) Anmeldetag: 19.03.2005
(51) Int. Cl.: A61F 13/02

(54) **MEDIZINISCHES PFLASTER ZUR ANWENDUNG AUF DER HAUT**
MEDICAL PLASTER FOR APPLICATION ON THE SKIN
PANSEMENT MEDICAL DESTINE A ETRE APPLIQUE SUR LA PEAU

(30) Priorität: 31.03.2004 DE 102004016591
(43) Veröffentlichungstag der Anmeldung: 20.12.2006
(73) Patentinhaber: Bayer Schering Pharma Aktiengesellschaft, 13353 Berlin (DE)
(72) Erfinder: BRACHT, Stefan, 16548 Glienicke Nordbahn (DE)
(86) Internationale Anmeldenummer: PCT/EP2005/002970
(87) Internationale Veröffentlichungsnummer: WO 2005/097021

(56) Entgegenhaltungen:
- EP-A- 0 614 652
- EP-A- 1 062 926
- DE-C- 743 775
- US-A- 4 664 106
- PATENT ABSTRACTS OF JAPAN Bd. 1997, Nr. 05, 30. Mai 1997 (1997-05-30) & JP 09 010256 A (SATO EISAKU), 14. Januar 1997 (1997-01-14)

## Beschreibung

### Technisches Gebiet

Die Erfindung betrifft ein medizinisches Pflaster zur Anwendung auf der Haut und ein Verfahren zu seiner Herstellung. Dieses System besteht aus einem haftklebenden Innenbereich, einen den Innenbereich umschließenden Außenbereich, wobei dieser nicht haftklebend ausgebildet.

### Stand der Technik

Es ist bekannt, dass medizinische Pflaster dem Tragen auf der Haut in der Regel einen deutlichen Schmutzrand hinterlassen. Dieses Phänomen verstärkt sich in Abhängigkeit von der Applikationsdauer und nimmt besonders problematische Formen an, wenn Pflaster länger als 3 und bis zu 7 Tagen getragen werden. Dieser Fall trifft insbesondere auf wirkstoffhaltige transdermale Pflaster zu, die im Bereich der Hormonsubstitution oder der hormonellen Kontrazeption häufig 7 Tage lang appliziert werden.

Der Schmutzrand bildet sich im Wesentlichen durch Anhaftung von Textilfasern, Schmutz- und Hautpartikeln an die am Rand offen liegende Schnittkante der Klebschicht. Je nach Grenzflächenenergien und Kleberbeschaffenheit können Partikel sogar durch Umfließen des Klebers in die Klebschicht aufgenommen werden. Besonders verstärkend kommt hinzu, dass sich Pflaster bei längerem Tragen auf der Haut durch mechanische Beanspruchung verschieben können, wobei zusätzlich am Rand Klebstoff austritt, der durch Anhaftung von Partikeln den Schmutzrand vergrößert. Die ebenfalls vom Rand her beginnende Ablösung der Pflaster liefert nach längerer Applikationszeit weitere Ablagerungsfläche für Schmutz.

Beim Entfernen der Pflaster bleiben mehr oder weniger große Teile des gebildeten Schmutzrandes auf der Haut zurück. Sie lassen sich mechanisch zumeist schlecht abreiben und verschwinden erst nach mehreren Tagen durch die übliche Körperpflege.

Zwar konnten durch Erhöhung der Kohäsion der medizinischen Haftkleber, beispielsweise durch Zumischung langkettiger Polymere oder durch chemische Q uervernetzung, einige Verbesserungen erzielt werden, jedoch wird mit erhöhter Kohäsion und verminderter Plastizität häufig auch die Langzeitklebefähigkeit vermindert. Nur ein partiell fließfähiger Haftkleber mit aggressivem Klebeverhalten kann das Kleben auf der Haut für bis zu einer Woche gewährleisten. In diesem Feld widersprüchlicher Anforderungen an den medizinischen Haftkleber ist es bis heute nicht möglich, die Bildung von kosmetisch störenden Schmutzrändern bei der Langzeitanwendung zu vermeiden.

Aus der Patentliteratur sind medizinische Pflastersysteme mit verschiedenartigen haftklebenden Bereichen bekannt.

Die US-Patentschrift US 4,664,106 beschreibt ein Wundpflaster, welches durch eine ablösbare Schutzfolie geschützt ist und einen klebstofffreien Randbereich besitzt, welcher bei Benutzung abgezogen wird.

Die US-Patentschrift US 5,690,610 beschreibt ein klebendes Kompressionsmaterial zur Blutstillung, wobei der Körper eine klebstofffreie druckplatte ist. Diese Druckplatte ist zwischen Kissen und hautberührender Klebstoffschicht angebracht und entspricht in seinen Abmessungen ebendieser Klebstoffschicht. Ein nicht haftklebender Bereich bei Benutzung ist nicht offenbart.

Die US-Patentschrift US 5,599,289 offenbart ein medizinisches Pflastersystem, welches über eine Trägerbahn, ein über dieser Trägerbahn liegendes Klebstoffmaterial und eine über dem Klebstoff liegende Trennlage verfügt. Ein nicht haftklebender Außenbereich bei Benutzung ist nicht offenbart.

Die DE-Patentschrift DE 743 775 beschreibt ein Wundpflaster mit Pflasterunterlage aus sterilem Material und einer Klebstoffschicht. Gazestreifen sind bis zur Benutzung so angeordnet, dass gleichzeitig Klebstoffschicht und sterile schicht geschützt sind. Die Patentschrift betrachtet ein nicht vollständiges Haftens des Pflasters und damit indirekt nicht haftklebende Randbereiche als Unsicherheit und Nachteil.

### Darstellung der Erfindung

Aufgabe der Erfindung ist deshalb, die erwähnten Nachteile der konventionellen medizinischen Pflaster, insbesondere das Problem der Schmutzrandbildung zu überwinden.

Erfindungsgemäß wird die Aufgabe durch ein medizinisches Pflaster zur Anwendung auf der Haut gelöst, bei welchem die Kontaktfläche zur Haut einen haftklebenden Innenbereich sowie einen den Innenbereich umschließenden Außenbereich aufweist. Dabei ist der Außenbereich an seiner hautseitig liegenden Oberfläche nicht haftklebend ausgebildet.

Für Pflaster lässt sich dies am zweckmäßigsten durch die Einbringung einer zusätzlichen Schicht in der Randzone des Pflasters realisieren, vorzugsweise durch eine am Rand integrierte schmale Folie, in der Folge als Randfolie bezeichnet. Diese Randfolie liegt bei der Anwendung zwischen der hautseitig haftklebenden Schicht des Pflasters und der Haut, wodurch der Kontakt des Klebers mit der Haut in dieser Zone unterbunden wird.

Im Falle von wirkstoffhaltigen Pflastern sollte die Randfolie vorzugsweise aus einem Material bestehen, das für den oder die enthaltenen Wirkstoffe praktisch undurchlässig ist. Andernfalls kommt es über die Randzone ebenfalls zu einer Wirkstoffabgabe des Pflasters an die Haut, die wegen des konstruktionsbedingt wenig reproduzierbaren Kontaktes der Randzone mit der Hautoberfläche und weiterhin wegen der über die Randfolie gegenüber der zentralen Klebschicht zwangsläufig mit abweichender Kinetik verlaufenden Wirkstoffabgabe unerwünscht ist. Aus diesen Gründen eignen sich für die Randfolie besonders die Materialien Polyethylenterephthalat (PET), Polyvinylchlorid (PVC), Polyvinylidenchlorid (PVDC) oder Polyvinylchlorid-Polyvinylidenchlorid-Copolymere (z.B. Saran^{®} ) und Polyacrylnitril (z.B. Barex^{®} ). PET (z.B. Hostaphan^{®} ) wird besonders bevorzugt wegen der Vielzahl kommerziell verfügbarer Typen, insbesondere im Bereich sehr dünner Folien.

Die je nach Sperrwirkung der Randfolie ggf. noch verbleibende Wirkstoffabgabe durch die Randzone liegt vorzugsweise um mindestens den Faktor 10 geringer, besonders bevorzugt um mindestens den Faktor 100 geringer als die Wirkstoffabgabe pro Fläche über den haftklebenden Bereich des Pflasters.

Für die Breite der vorzusehenden Randzone wurde ein optimaler Bereich ermittelt: zu große Breiten führen zu einem Rand, der sehr leicht durch mechanischen Kontakt mit der Kleidung oder anderen Fremdkörpern umgebogen und mitgezogen wird, wodurch es zur vorzeitigen Ablösung des Pflasters kommen kann. Ein zu schmaler Rand ist dagegen einerseits verfahrenstechnisch nur schwer mit ausreichender Symmetrie und Reproduzierbarkeit zu realisieren und bietet andererseits im Verhältnis zum Produktionsaufwand nur noch ungenügenden Schutz der innenliegenden Kleberfläche gegen Partikelkontamination von außen.

Es wurde gefunden, dass die Randbreite im Bereich von 0,5 bis 5 mm liegen sollte, vorzugsweise im Bereich von 0,75 bis 3 mm und besonders bevorzugt im Bereich von 1 bis 1,5 mm. Der Optimalwert hängt jedoch auch von der Größe des Pflasters und den Krümmungsradien seiner Konturlinie ab: bei größeren Pflastern mit weiteren Radien können auch breitere Ränder vorgesehen werden. Der Idealwert von 1-1,5 mm Breite gilt insbesondere für Pflaster in einer Größe von 5 bis 25 cm² von runder, ovaler oder viereckig abgerundeter Form.

In Einzelfällen kann es sinnvoll sein, die Breite der Randzone entlang der Konturlinie des Pflasters variabel zu gestalten, z.B. breiter im Bereich geringer Krümmungsradien, schmaler bei engen Radien, da an engen Krümmungen die Gefahr des mechanischen Umklappens des Randes bei Kontakt mit Kleidung besonders groß ist.

Hinsichtlich der Dicke der Randfolie wurde ebenfalls ein Optimum zwischen zu dünnem und zu dickem Material gefunden: Eine zu dicke Folie verringert je nach Material durch ihre Starrheit selbst bei geringer Breite schon den Tragekomfort des Pflasters durch mechanische Spannungen auf der Haut.

Eine zu dünne Folie ergibt dagegen keine ausreichende Verstärkung des Randes, so dass die Randzone leicht den formschlüssigen Kontakt mit der Haut verliert und z.B. durch Umknicken oder Einrollen am Rand des Pflasters hoch steht.

Ein Material von geeigneter Dicke gibt zwar einerseits der Randzone die nötige Formhaltigkeit, beeinträchtigt aber noch nicht spürbar den mechanischen Tragekomfort auf der Haut.

Erfindungsgemäß kann der nicht haftklebende Außenbereich eine auf die Klebschicht des Pflasters aufgebrachte Umrandung aus einer Kunststofffolie oder einem Kunststofffilm darstellen.

Im Falle von Poylesterfolien (PET) wurde eine Dicke von 6 bis 150µm, vorzugsweise 9 bis 75 µm und besonders bevorzugt etwa 36 µm als geeignet ermittelt.

Handelt es sich bei der Schicht um einen Polymerfilm auf der Basis eines Kohlenwasserstoff- oder Silikonpolymers so kann dieser eine Schichtdicke von 6 bis 150 µm, vorzugsweise 15 bis 75 µm besitzen.

Auch kann die aufgebrachte Umrandung in der Fläche teilweise oder vollständig über die Konturlinien des restlichen Pflasters hinausragen.

Erfindungsgemäß kann der nicht haftklebende Außenbereich entlang der Konturlinie des Pflasters eine Breite von 0,5 bis 5,0 mm aufweisen, vorzugsweise 1 bis 3 mm und besonders bevorzugt 1,0 bis 1,5 mm.

Es wurde weiterhin gefunden, dass sich eine abweisende Beschichtung der Randfolie an der der Haut zugewandten Oberfläche vorteilhaft auswirkt, indem sie ein Einfließen oder Unterkriechen des Haftklebers während der Lagerung und während der Applikation auf der Haut unterdrückt. Die Randzone bleibt dadurch länger frei von Kleber, und die Bildung von Schmutzrändern wird weiter reduziert.

Im Falle von Haftklebern auf der Basis von Poylacrylaten oder Kohlenwasserstoffen (z.B. Poylisobutylen) eignen sich Silikonisierungen als abweisende Beschichtungen, während bei Haftklebern auf Silikonbasis die Beschichtung auf der Basis von speziellen, fluorierten Polymeren erfolgen sollte.

Bei Pflastern für eine lange Applikationsdauer von 3 bis 7 Tagen sollte diese abweisende Beschichtung einen geringen mechanischen Abrieb zeigen, um eine lange Haltbarkeit zu gewährleisten. Die Problematik des Abriebs ist dem Fachmann auf dem Gebiet der Silikonisierung bekannt und verschiedene Techniken und Produkte stehen zur Reduktion desselben zur Verfügung.

Um die erfindungsgemäße Technologie einer schmutzminimierenden Randzone für den Anwender besser erkennbar zu machen, kann es vorteilhaft sein, die Randzone hinsichtlich Farbe oder Transparenz anders zu gestalten als die übrige Fläche des Pflasters. Dies kann z.B. durch Lackierung oder Metallisierung der Randfolie geschehen, vorzugsweise durch Aluminiumbedampfung der der Haut abgewandten Oberfläche der Randfolie. Die visuelle Hervorhebung der Randzone kann alternativ auch durch partielle Bedruckung der Rückschicht des Pflasters erfolgen.

Es können im erfindungsgemäßen medizinischen Pflaster mindestens ein pharmazeutischer Wirkstoff enthalten sein.

Die Wirkstoffabgaberate pro Fläche im Außenbereich kann um mindestens den Faktor 10 geringer sein als die Abgaberate im wirkstoffhaltigen Innenbereich, vorzugsweise mindestens um den Faktor 100.

Auch kann das medizinische Pflaster Wirkstoffe zur Hormonersatztherapie oder zur transdermalen Kontrazeption enthalten.

Vorteilhafte Ausgestaltungen für Wirkstoffe sind dabei Gestagene und Estrogene, vorzugsweise Gestoden in Kombination mit einem Estrogen.

Auch kann die die Anwendungsdauer für das medizinische Pflaster 3 bis 7 Tage betragen.

Für die Rückschicht des erfindungsgemäßen medizinischen Pflasters ergeben sich keine über die auf diesem Gebiet üblichen Anforderungen hinausgehenden Notwendigkeiten. Bevorzugte Verwendung finden Polyesterfolien (PET, z.B. Hostaphan® ), Polyethylenfolien (z.B. CoTran 9720) oder Mehrschichtlaminate, die diese Materialien enthalten.

Die Klebschicht kann aus einer oder mehreren Schichten bestehen, die der Zusammensetzung nach identisch oder verschieden sein können. Bevorzugt werden ein- oder zweischichtige Systeme die in einer oder in beiden Schichten mindestens einen pharmazeutischen Wirkstoff enthalten. Die Erfindung betrifft jedoch ausdrücklich auch nicht wirkstoffhaltige medizinische Pflaster z.B. aus dem Bereich der Wundversorgung, Katheterfixierung oder Wundheilungsförderung.

Als haftklebende Formulierungen eignen sich drucksensitive Kleber auf der Basis von Poylacrylaten, Kohlenwasserstoffen oder Silikonen sowie Mischungen davon, wie sie dem Fachmann auf dem Gebiet medizinischer Pflaster und transdermaler therapeutischer Systeme bekannt sind.

Wegen der erfindungsgemäß unterdrückten Bildung von Schmutzrändern können allerdings auch wenig kohäsive und stark adhäsive Formulierungen zum Einsatz kommen, die ansonsten wegen des Austritts von Klebermasse am Pflasterrand während Lagerung und/oder Applikation nicht akzeptabel sind. Solche Formulierungen basieren z.B. auf unvernetzten Poylacrylathaftklebern (z.B. Durotak 387-2051, Durotak 387-2287), unvernetzten Silikonhaftklebern mit hoher Spontanklebrigkeit/Tack (z.B. Dow Corning Bio PSA 430X oder 460X mit X=1,2,3) oder Kohlenwasserstoffklebern mit einem hohen Anteil an niedermolekularen Kohlenwasserstoffen (z.B. mehr als 20% Oppanol B10).

Der wirkstoffhaltige Teil des Pflasters kann weiterhin auch in Art eines Reservoirsystems mit einem halbfesten oder flüssigen Wirkstoffreservoir ausgeführt werden.

Als wieder ablösbare Schutzfolie eignen sich alle für medizinische Pflaster und transdermale therapeutische Systeme üblichen Folien und beschichtete Papiere.

Diese Schutzfolie kann in gleicher Größe und Kontur wie das aufliegende Pflaster ausgeführt werden oder auch seitlich überstehend ausgebildet sein. Zur Erleichterung der Applikation kann eine Applizierhilfe in der Schutzfolie eingestanzt werden.

Es wurde gefunden, dass die Bildung von Schmutzrändern kann in sehr hohem Maße, teilweise sogar vollständig vermieden werden, wenn die Randzone des medizinischen Pflastern nicht haftklebend ausgerüstet ist. Durch einen allseits am Rand vorgesehenen, kleberfreien Rand wird der weiter innen liegende Rand der Klebfläche weitestgehend vor Schmutz und Textilienkontakt geschützt.

Es ist zu bemerken, dass dieser Schutz des Klebschichtrandes in Verbindung mit einer mechanischen Verstärkung dieser Randzone überraschenderweise sogar zur Verbesserung der Langzeitklebefähigkeit des Pflasters führt, indem das vom Rand durch Kantenabhebung und Reiben an Kleidung bedingte mechanische Ablösen des Pflasters vermindert bzw. verzögert wird.

Ursprünglich war hier zu erwarten, dass ein am Rand nicht klebendes Pflaster sogar besonders schnell durch Kontakt mit Kleidung oder sonstige mechanische Beanspruchung abgerieben wird.

### Beschreibung der Abbildungen:

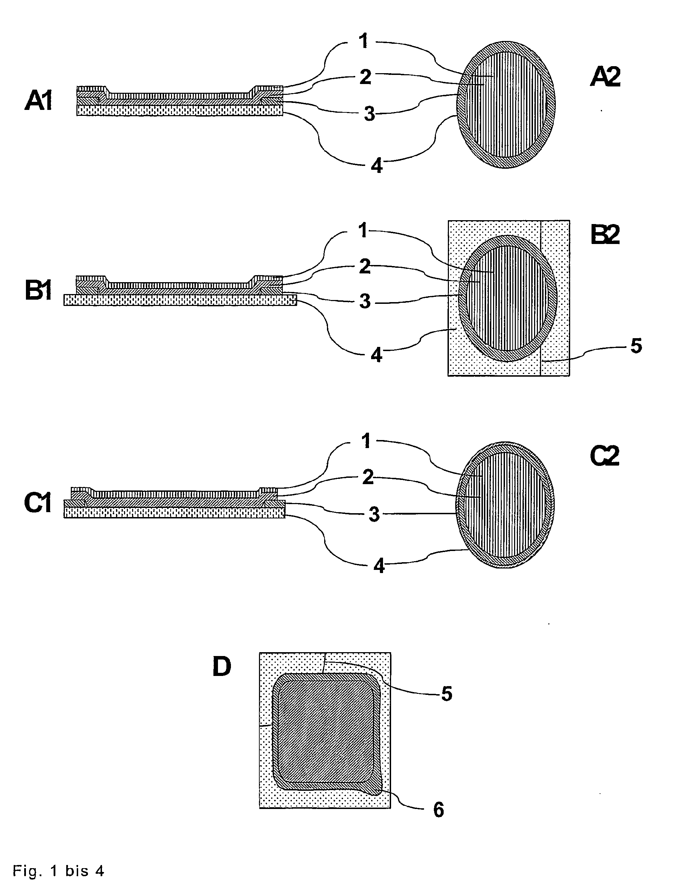

Die Abbildungen Figur 1 - A1 (Querschnitt) und A2 (Draufsicht) zeigen ein erfindungsgemäßes medizinisches Pflaster in der einfachsten Anordnung aller Komponenten, das für den Zweck dieser Erfindung als Typ A bezeichnet wird. Auf eine Rückschicht (1) folgt mindestens eine, ggf. wirkstoffhaltige Matrixschicht (2), die am Rand von einer Randfolie (3) gefolgt wird, wobei abschließend eine wieder ablösbare Schutzfolie (Schutzfolie) vorgesehen ist. Diese Schutzfolie (4) steht am Rand nicht über, es handelt sich um ein vollständig durchgestanztes System.

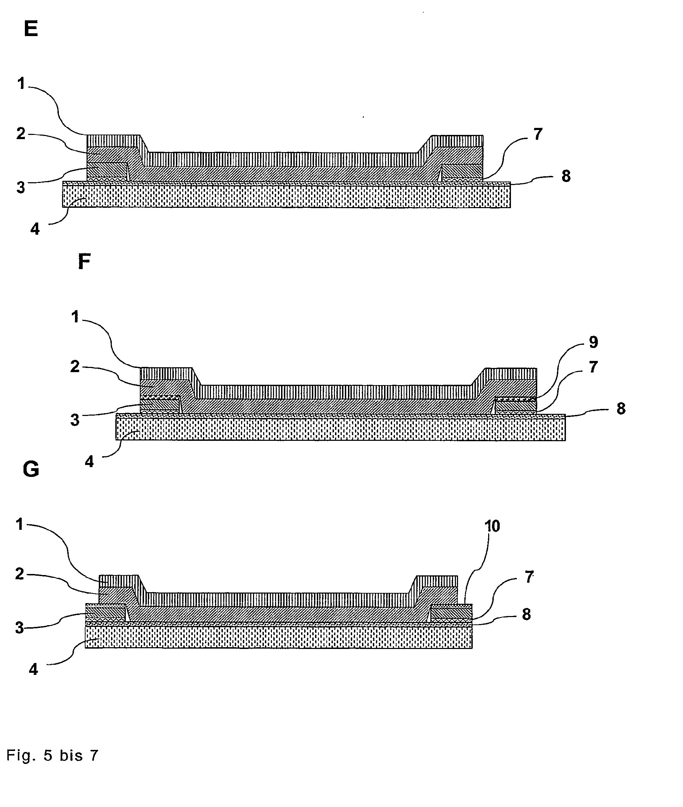

Die Abbildungen Figur 2 - B1 (Querschnitt) und B2 (Draufsicht) zeigen ein erfindungsgemäß als Typ B bezeichnetes medizinisches Pflaster, bei dem im Unterschied zu Typ A die Schutzfolie (4) am Rand übersteht.

Die Abbildungen Figur 3 - C1 (Querschnitt) und C2 (Draufsicht) zeigen ein erfindungsgemäß als Typ C bezeichnetes medizinisches Pflaster, bei dem die äußere Konturlinie der Randfolie (3) außerhalb der Konturlinie des Innenbereiches, bestehend aus Rückschicht (1) und Matrixschicht (2), verläuft.

In Abbildung Figur 4 - D werden weitere, optionale Ausgestaltungsmerkmale der erfindungsgemäßen medizinischen Pflaster dargestellt, beispielhaft am Typ B:

Für die leichtere Applizierbarkeit ist ein Schnitt (5) in der Schutzfolie vorgesehen, entlang dessen zunächst nur ein Teil der Schutzfolie (4) entfernt werden kann, bevor das Pflaster teilweise aufgeklebt, dann die zweite Hälfte der Schutzfolie (4) entfernt und das Pflaster abschließend vollflächig aufgeklebt wird. Zur Erleichterung der späteren Entfernung des Pflasters ist an dem nicht klebenden Randbereich eine laschenartige Vergrößerung (6) vorgesehen. An diesem Ende kann das Pflaster am Ende der Applikationszeit leichter angefasst und abgezogen werden.

Die Abbildung Figur 5 - E zeigt Details einer bevorzugten Ausführung des Pflasters vom Typ A:

Die zur Schutzfolie (4) hin liegende Oberfläche der Randfolie (3) weist eine gegenüber der Klebermatrix (2) abweisende Beschichtung (7) auf. Die zum Pflaster weisende Oberfläche der Schutzfolie (4) ist notwendigerweise ebenfalls abweisend beschichtet , wobei diese Beschichtung (8) identisch oder abweichend von der Beschichturtg(7) der Randfolie (3) ausgeführt sein kann.

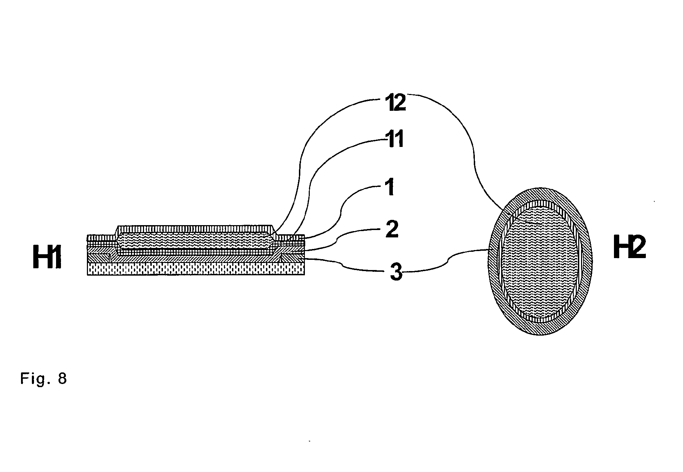

Die Abbildung Figur 6 - F zeigt Details einer weiteren bevorzugten Ausführung des Pflasters vom Typ A:

Die zur Schutzfolie (4) hin liegende Oberfläche der Randfolie (3) weist eine gegenüber der Klebermatrix (2) abweisende Beschichtung (7) auf. Weiterhin weist die zur Matrixschicht (2) hin liegende Oberfläche der Randfolie (3) eine Aluminisierung (9) zum Zweck der leichten visuellen Unterscheidbarkeit der Randzone vom übrigen Pflaster auf.
Die Abbildung Figur 7 - G zeigt Details einer bevorzugten Ausführung des Pflasters vom Typ C:

Die zur Schutzfolie (4) hin liegende Oberfläche der Randfolie (3) weist eine gegenüber der Klebermatrix (2) abweisende Beschichtung (7) auf. Weiterhin weist auch die zur Matrixschicht (2) hin liegende Oberfläche der Randfolie (3) einen abweisende Beschichtung (10) auf. Die zum Pflaster weisende Oberfläche der Schutzfolie (4) ist notwendigerweise wiederum abweisend beschichtet (8), wobei diese Beschichtung (8) identisch oder abweichend von den Beschichtungen (7 und 10) der Randfolie (3) ausgeführt sein kann. Die Beschichtungen (7 und 10) der Randfolie (3) können gleich oder verschieden ausgeführt sein. Die Trennkraft der klebenden Matrixschicht (2) von der Beschichtung (8) der Schutzfolie (4) ist vorzugsweise größer als die Trennkraft der klebenden Matrixschicht (2) von der Beschichtung (10) der Randfolie (3), damit im Herstellverfahren die klebende Matrixschicht (2) von der Beschichtung (10) der Randfolie (3) abgezogen werden kann, ohne dass sie sich auch von der Beschichtung (8) der Schutzfolie (4) löst.

Die Abbildungen Figur 8 - H1 (Querschnitt) und H2 (Draufsicht) zeigen die Übertragung des erfindungsgemäßen Prinzips auf wirkstoffhaltige Pflaster vom Typ eines Reservoirsystems. Zwischen der Rückschicht (1) und einer heißsiegelfähigen Zwischenschicht (11), die auch als eine Steuermembran für die Wirkstoffabgabe ausgeführt sein kann, befindet sich allseitig eingeschlossen ein flüssiges oder halbfestes Wirkstoffreservoir (12). Ansonsten entspricht dieses System dem erfindungsgemäßen Typ A. Eine überstehende Schutzfolie (4) kann analog zu Typ B vorgesehen werden.

Für die Herstellung von Pflastern mit einer am Rand nicht klebenden Zone wird verfahrenstechnisch die Einbringung einer Randfolie entlang des Pflasterrandes bevorzugt. Im folgenden werden die wesentlichen Herstellschritte für die Basistypen A, B und C beschrieben, von denen sich die Typen D bis G Variation der Ausgangsmaterialien bzw. der Verfahrensschritte ableiten lassen.

Ein Verfahren zur Herstellung des erfindungsgemäßen medizinischen Pflasters vom Typ A, umfassend die Herstellung eines Laminates für den Innenbereich bestehend aus einer Rückschicht, mindestens einer wirkstoffhaltigen Haftkleberschicht und für den Außenbereich vorzugsweise einer wieder abziehbaren Schutzfolie sowie die Verwendung einer vorzugsweise einseitig abweisend beschichteten Folie, umfasst folgenden Schritte :
a) in einem Schritt werden in die für den Randbereich vorgesehene Folie den Konturen des späteren Innenbereiches entsprechende Ausstanzungen vorgenommen, und die ausgestanzten Areale werden verworfen,
b) in einem weiteren Schritt wird die unter Schritt a) ausgestanzte Folie mit dem Schutzfolie der herzustellenden Pflaster übereinander liegend zusammengeführt, wobei im Falle einer abweisend beschichteten Oberfläche diese abweisende Oberfläche der gestanzten Folie auf der ebenfalls abweisend beschichteten Oberfläche der Schutzfolie zu liegen kommt,
c) in einem weiteren Schritt wird das Laminat des Innenbereiches, bestehend aus mindestens einer haftklebenden Matrixschicht sowie der Rückschicht, von dem zuvor eine ggf. im Prozess der Herstellung vorgesehene Schutzfolie entfernt wurde, mit der haftklebenden Seite auf den Verbund von gestanzter Folie des Randbereiches und der Schutzfolie aufkaschiert, wobei die haftklebende Seite des Laminates auf die unbeschichtete Oberfläche der Folie des Randbereiches und über die Ausstanzungen darin direkt auch mit der abweisend beschichteten Oberfläche der Schutzfolie in flächige Verbindung gebracht wird,
d) in einem letzten Schritt wird die Stanzung der äußeren Konturlinie des Pflasters durch alle Schichten des gebildeten Laminates hindurch vorgenommen, wobei diese äußere Konturlinie in dem für die Breite der Randzone vorgesehenen Abstand um die unter Schritt a) vorgenommenen Ausstanzungen in der Folie des Randbereiches herum verläuft.

Ein Verfahren zur Herstellung des erfindungsgemäßen medizinischen Pflasters vom Typ B umfassend die Verfahrensschritte, wie vorher angegeben, ist verändert dadurch, dass das Laminat in Schritt d) abweichend nicht durch alle Schichten hindurch gestanzt wird, sondern die Konturlinie des Pflasters durch alle Schichten außer der Schutzfolie gestanzt wird. Danach wird das überschüssige Laminatgitter von der Schutzfolie abgezogen und verworfen wird, bevor in einem weiteren Schritt eine über den Rand des Pflaster hinausragende Kontur der Schutzfolie gestanzt oder geschnitten wird.

Ein Verfahren zur Herstellung des erfindungsgemäßen medizinischen Pflasters vom Typ C, umfassend die Herstellung eines Laminates für den Innenbereich bestehend aus einer Rückschicht, mindestens einer wirkstoffhaltigen Haftkleberschicht und vorzugsweise einer wiederabziehbaren Schutzfolie sowie die Verwendung einer mindestens einseitig abweisend beschichteten Folie für den Außenbereich, ist dadurch gekennzeichnet, dass es die folgenden Schritte umfasst:
a) in einem Schritt werden in die für den Randbereich vorgesehene, mindestens einseitig abweisend beschichtete Folie den Konturen des späteren Innenbereiches entsprechende Ausstanzungen vorgenommen, und die ausgestanzten Areale werden verworfen,
b) in einem weiteren Schritt wird die unter Schritt a) ausgestanzte Folie mit dem Schutzfolie der herzustellenden Pflaster übereinander liegend zusammengeführt, wobei die mindestens eine abweisend beschichtete Oberfläche der gestanzten Folie auf der dem Schutzfolie abgewandten Seite zu liegen kommt,
c) in einem weiteren Schritt wird das Laminat des Innenbereiches, bestehend aus der Rückschicht sowie mindestens einer haftklebenden Matrixschicht, von dem zuvor eine ggf. im Herstellprozess vorgesehene Schutzfolie entfernt wurde, mit der haftklebenden Seite auf den Verbund von gestanzter Folie des Randbereiches und dem Schutzfolie aufkaschiert, wobei die haftklebende Seite des Laminates auf die unbeschichtete Oberfläche der Folie des Randbereiches und über die Ausstanzungen darin direkt auch mit der abweisend beschichteten Oberfläche des Schutzfolies in flächige Verbindung gebracht wird,
d) in einem weiteren Schritt wird die Stanzung der Konturlinie des Innenbereiches des Pflasters durch die Rückschicht und die mindestens eine Matrixschicht hindurch vorgenommen, wobei diese innere Konturlinie in dem vorgesehenen Abstand um die unter Schritt a) vorgenommenen Ausstanzungen in der Folie des Randbereiches herum verläuft,
e) In einem weiteren Schritt wird das überschüssige Laminatgitter bestehend aus Rückschicht und mindestens einer Matrixschicht abgezogen und verworfen,
f) in einem letzten Schritt wird die Stanzung der äußeren Konturlinie des Pflasters durch den Außenbereich und den Schutzfolie hindurch vorgenommen, wobei diese äußere Konturlinie in dem für die Breite der überstehenden Randzone vorgesehenen Abstand um die unter Schritt d) vorgenommenen Ausstanzungen in der Folie des Randbereiches herum verläuft.

**Ausführungsbeispiel**

| **Pflasterzusammensetzung:** | |
|---|---|
| Pflasterrückschicht | |
| Polyethylenfolie CoTran 9720 (Fa. 3M) | |
| Haftklebermatrix; Schichtstärke 100 g/m² | Teile (getrocknet) |
| Ethinylestradiol | 0,6 |
| Gestoden | 1,9 |
| MA73A | 97,5 |
| Gesamt | 100,0 |
| Randfolie | |

Polyethylenterephthalat (PET) 36 µm (Hostaphan RN 36) einseitig silikonisiert (Fa. Laufenberg).
Schutzfolie
ScotchPak 9742 (Fa. 3M) = 117 µm Polyesterfolie, einseitig fluoropolymerbeschichtet

Die Herstellung des Beispielsystems beginnt mit der wirkstoffhaltigen Haftklebermatrix. Dazu werden die beiden Wirkstoffe Ethinylestradiol und Gestagen in Haftkleberlösung MA73A (Fa. Adhesives Research, Haftkleber auf der Basis von Polyisobutylen mit einem Klebharzzusatz auf der Basis von hydrierten Kolophoniumestern) aufgelöst.

Diese Lösung wird mit einem Handfilmziehrahmen in einer Schichtdicke von 500 µm auf eine silikonisierte Polyesterfolie (Schutzfolie) beschichtet und nach 10 Minuten Trocknung in einem Laborabzug bei Raumtemperatur noch weitere 30 Minuten bei 60°C in einem Trockenschrank getrocknet.

Der getrocknete Film weist eine Schichtstärke von ca. 100 g/m² auf, ggf. muss die Spalthöhe bei der Beschichtung angepasst werden, bis das angestrebte Flächengewicht getroffen wird.

Der getrocknete Film wird mit der Rückschicht aus CoTran 9720 abgedeckt.

In die Randfolie werden kreisrunde Ausstanzungen von 10 cm² Fläche vorgenommen.

Die Randfolie wird anschließend mit der silikonisierten Seite nach unten auf die Schutzfolie Folie gelegt, wobei die silikonisierte Seite der Schutzfolie nach oben weist.

Sodann wird von der hergestellten Klebermatrix die Schutzfolie abgezogen und die offen liegende Kleberfläche wird auf die Randfolie kaschiert. Die Kleberschicht geht dabei mit der unsilikonisierten Oberfläche der Randfolie eine dauerhafte Verbindung ein; durch die Ausstanzungen in der Randfolie kommt die Kleberschicht weiterhin mit der silikonisierten Oberfläche der Schutzfolie in eine leicht wieder lösbare Verbindung.

Abschließend wird aus dem Verbund ein 11,5 cm² großes, rundes TTS ausgestanzt, wobei die Stanzung dieser äußeren Kontur symmetrisch um die runde Ausstanzung von 10 cm² in der Randfolie herum gelegt wird.

Dabei entsteht eine Randzone von ca. 1 mm Breite.

## Patentansprüche

1. Medizinisches Pflaster zur Anwendung auf der Haut, bestehend aus einer Rückschicht, mindestens einer wirkstoffhaltigen Haftkleberschicht und vorzugsweise einer wiederabziehbaren Schutzfolie, wobei die Kontaktfläche zur Haut einen haftklebenden Innenbereich aufweist sowie einen nicht haftklebenden Außenbereich,
**dadurch gekennzeichnet, dass** der Außenbereich eine den Innenbereich umschließende mechanische Verstärkung in Form einer zusätzlichen Schicht im Aufbau des Pflasters aufweist.

2. Medizinisches Pflaster nach Anspruch 1,
**dadurch gekennzeichnet, dass** es sich bei der bezeichneten Schicht um eine Polyesterfolie in einer Schichtdicke von 6 bis 150 µm , bevorzugt 15 bis 75 µm, handelt.

3. Medizinisches Pflaster nach Anspruch 1 und 2
**dadurch gekennzeichnet, dass** sich bei der bezeichneten Schicht um einen Polymerfilm auf der Basis eines Kohlenwasserstoff- oder Silikonpolymers in einer Schichtdicke von 6 bis 150 µm, bevorzugt 15 bis 75 µm, handelt.

4. Medizinisches Pflaster nach einem oder mehreren der vorangehenden Ansprüche,
**dadurch gekennzeichnet, dass** der nicht haftklebende Außenbereich entlang der Konturlinie des Pflasters eine Breite von 0,5 bis 5 mm, bevorzugt 1,0 bis 3,0 mm , besonders bevorzugt 1,0 bis 1,5 mm, aufweist.

5. Medizinisches Pflaster nach einem oder mehreren der vorangehenden Ansprüche ,
**dadurch gekennzeichnet, dass** der nicht haftklebende Außenbereich eine auf die Klebschicht des Pflasters aufgebrachte Umrandung aus einer Kunststofffolie oder einem Kunststofffilm darstellt.

6. Medizinisches Pflaster nach einem oder mehreren der vorangehenden Ansprüche;
**dadurch gekennzeichnet, dass** die aufgebrachte Umrandung in der Fläche teilweise oder vollständig über die Konturlinien des restlichen Pflasters hinausragt.

7. Medizinisches Pflaster nach einem oder mehreren der vorangehenden Ansprüche,
**dadurch gekennzeichnet, dass** die hautseitig liegende Oberfläche des Außenbereiches eine den Haftkleber abweisende Beschichtung aufweist, bevorzugt eine Silikonisierung oder eine Beschichtung mit einem fluorhaltigen Poylmer.

8. Medizinisches Pflaster nach einem oder mehreren der vorangehenden Ansprüche,
**dadurch gekennzeichnet, dass** der nicht haftklebende Außenbereich nach Farbe oder Transparenz gegenüber der restlichen Fläche abweichend ausgeführt ist, bevorzugt durch eine Metallbedampfung einer der am Schichtaufbau der Randzone beteiligten Folien.

9. Medizinisches Pflaster nach einem oder mehreren der vorangehenden Ansprüche,
**dadurch gekennzeichnet, dass** mindestens ein pharmazeutischer Wirkstoff enthalten ist.

10. Medizinisches Pflaster nach einem oder mehreren der vorangehenden Ansprüche,
**dadurch gekennzeichnet, dass** die Wirkstoffabgaberate pro Fläche- im Außenbereich um mindestens den Faktor 10 geringer ist als die Abgaberate im wirkstoffhaltigen Innenbereich, bevorzugt mindestens um den Faktor 100.

11. Medizinisches Pflaster nach einem oder mehreren der vorangehenden Ansprüche,
**dadurch gekennzeichnet, dass** das Pflaster 3 bis 7 Tage appliziert bleibt.

12. Medizinisches Pflaster nach einem oder mehreren der vorangehenden Ansprüche,
**dadurch gekennzeichnet, dass** es Wirkstoffe zur Hormonersatztherapie oder zur transdermalen Kontrazeption enthält.

13. Medizinisches Pflaster nach einem oder mehreren der vorangehenden Ansprüche, ..
**dadurch gekennzeichnet, dass** der Wirkstoff ein Gestagen und/oder ein Estrogen ist.

14. Medizinisches Pflaster nach einem oder mehreren der vorangehenden Ansprüche,
**dadurch gekennzeichnet, dass** es einen Gehalt an Gestoden in Kombination mit einem Estrogen aufweist.

15. Verfahren zur Herstellung des medizinischen Pflasters nach Anspruch 1, umfassend die Herstellung eines Laminates für den Innenbereich bestehend aus einer Rückschicht, mindestens einer wirkstoffhaltigen Haftkleberschicht und vorzugsweise einer wiederabziehbaren Schutzfolie sowie die Verwendung einer einseitig abweisend beschichteten Folie für den Außenbereich, **dadurch gekennzeichnet, dass** es die folgenden Schritte umfasst:
a) in einem Schritt werden in die für den Randbereich vorgesehene Folie den Konturen des späteren Innenbereiches entsprechende Ausstanzungen vorgenommen, und die ausgestanzten Areale werden verworfen,
b) in einem weiteren Schritt wird die unter Schritt a) ausgestanzte Folie mit der Schutzfolie der herzustellenden Pflaster übereinanderliegend zusammengeführt, wobei im Falle einer abweisend beschichteten Oberfläche diese abweisende Oberfäche der gestanzten Folie auf der ebenfalls abweisend beschichteten Oberfläche der Schutzfolie zu liegen kommt,
c) in einem weiteren Schritt wird das Laminat des Innenbereiches, bestehend aus mindestens einer haftklebenden Matrixschicht sowie der Rückschicht, von dem zuvor eine gegebenenfalls im Herstellprozess vorgesehene Schutzfolie - entfernt wurde, mit der haftklebenden Seite auf den Verbund von gestanzter Folie des Randbereiches und der Schutzfolie aufkaschiert, wobei die haftklebende Seite des Laminates auf die unbeschichtete Oberfläche der Folie des Randbereiches und über die Ausstanzungen darin direkt auch mit der abweisend beschichteten Oberfläche der Schutzfolie in flächige Verbindung gebracht wird,
d) in einem letzten Schritt wird die Stanzung der äußeren Konturlinie des Pflasters durch alle Schichten des gebildeten Laminates hindurch vorgenommen, wobei diese äußere Konturlinie in dem für die Breite der Randzone vorgesehenen Abstand um die unter Schritt a) vorgenommenen Ausstanzungen in der Folie des Randbereiches herum verläuft.

16. Verfahren zur Herstellung des medizinische Pflasters nach Anspruch 8 umfassend die Verfahrensschritte nach Anspruch 15,
**dadurch gekennzeichnet, dass** das Laminat in Schritt
d) abweichend nicht durch alle Schichten hindurch gestanzt wird, sondern die Konturlinie des Pflasters durch alle Schichten, ausgenommen die Schutzfolie, gestanzt wird, wonach das überschüssige Laminatgitter von der Schutzfolie abgezogen und verworfen wird, bevor in einem weiteren Schritt eine über den Rand des Pflaster hinausragende Kontur der Schutzfolie gestanzt oder geschnitten wird.

## Claims

1. Medical patch for use on the skin, composed of a backing layer, at least one active substance layer of pressure-sensitive adhesive and, preferably, a removable protective film, the contact area to the skin having a pressure-sensitive adhesive interior and also an exterior which is not pressure-sensitive adhesive, **characterized in that** the exterior has a mechanical reinforcement surrounding the interior in the form of an additional layer in the construction of the patch.

2. Medical patch according to Claim 1, **characterized in that** the layer referred to comprises a polyester film in a layer thickness of from 6 to 150 µm, preferably 15 to 75 µm.

3. Medical patch according to Claim 1 and 2, **characterized in that** the layer referred to comprises a polymer film based on a hydrocarbon polymer or silicone polymer in a layer thickness of from 6 to 150 µm, preferably 15 to 75 µm.

4. Medical patch according to one or more of the preceding claims, **characterized in that** the non-adhesive exterior has a width along the contour line of the patch of from 0.5 to 5 mm, preferably 1.0 to 3.0 mm, with particular preference 1.0 to 1.5 mm.

5. Medical patch according to one or more of the preceding claims, **characterized in that** the non-adhesive exterior constitutes a border composed of a polymeric film or a polymeric sheet and applied to the adhesive layer of the patch.

6. Medical patch according to one or more of the preceding claims, **characterized in that** the applied border protrudes, in terms of its area, partly or completely beyond the contour lines of the rest of the patch.

7. Medical patch according to one or more of the preceding claims, **characterized in that** the skin-facing surface of the exterior has a coating which is repellent to the pressure-sensitive adhesive, preferably a siliconization or a coating with a fluorine-containing polymer.

8. Medical patch according to one or more of the preceding claims, **characterized in that** the non-adhesive exterior is implemented differently in colour or transparency from the remaining area, preferably by means of vapour deposition of metal on one of the films forming part of the layer construction of the marginal zone.

9. Medical patch according to one or more of the preceding claims, **characterized in that** at least one active pharmaceutical substance is present.

10. Medical patch according to one or more of the preceding claims, **characterized in that** the rate of active substance delivery per unit area in the exterior is lower by a factor of at least 10 than the rate of delivery in the active substance interior, with preference by a factor of at least 100.

11. Medical patch according to one or more of the preceding claims, **characterized in that** the patch remains applied for 3 to 7 days.

12. Medical patch according to one or more of the preceding claims, **characterized in that** it comprises active substances for hormone replacement therapy or for transdermal contraception.

13. Medical patch according to one or more of the preceding claims, **characterized in that** the active substance is a progestogen and/or an oestrogen.

14. Medical patch according to one or more of the preceding claims, **characterized in that** it comprises gestodene in combination with an oestrogen.

15. Process for producing the medical patch according to Claim 1, comprising the production of a laminate for the interior, composed of a backing layer, at least one active substance layer of pressure-sensitive adhesive and, preferably, a removable protective film, and also the use of a film repellently coated on one side for the exterior, **characterized in that** it comprises the following steps:
a) in one step diecuts corresponding to the contours of the subsequent interior are made in the film provided for the marginal region, and the diecut areas are discarded,
b) in a further step the film diecut under step a) is brought together one atop the other with the protective film of the patches that are to be produced; in the case of a repellently coated surface this repellent surface of the punched film comes to lie on the likewise repellently coated surface of the protective film,
c) in a further step the laminate of the interior, composed of at least one pressure-sensitive adhesive matrix layer and also the backing layer, from which any protective film envisaged in the production operation has been removed, is laminated by its pressure-sensitive adhesive side onto the assembly consisting of the punched film of the marginal region and the protective film, the pressure-sensitive adhesive side of the laminate being brought into two-dimensional communication with the uncoated surface of the film of the marginal region and, via the diecuts therein, directly with the repellently coated surface of the protective film as well,
d) in a last step the outer contour line of the patch is punched through all of the layers of the resultant laminate, this outer contour line extending at the distance intended for the width of the marginal zone round the diecuts, made under step a), in the film of the marginal region.

16. Process for producing the medical patch according to Claim 8, comprising the process steps according to Claim 15, **characterized in that** in step d), differently, the laminate is not punched through all of the layers but instead the contour line of the patch is punched through all of the layers with the exception of the protective film, whereafter the excess laminate mesh is removed from the protective film and discarded, before in a further step a protective film contour protruding beyond the margin of the patch is punched or cut.

## Revendications

1. Pansement médical destiné à être utilisé sur la peau, constitué par une couche arrière, au moins une couche auto-adhésive contenant une substance active et de préférence une feuille de protection pouvant être retirée, la surface de contact avec la peau présentant une zone interne auto-adhésive ainsi qu'une zone externe non adhésive, **caractérisé en ce que** la zone externe présente un renforcement mécanique entourant la zone interne sous forme d'une couche supplémentaire dans la structure du pansement.

2. Pansement médical selon la revendication 1, **caractérisé en ce qu'**il s'agit, pour la couche en question, d'une feuille en polyester présentant une épaisseur de couche de 6 à 150 µm, de préférence de 15 à 75 µm.

3. Pansement médical selon la revendication 1 et 2 **caractérisé en ce qu'**il s'agit, pour la couche en question, d'un film polymère à base d'un polymère hydrocarboné ou siliconé en une épaisseur de couche de 6 à 150 µm, de préférence de 15 à 75 µm.

4. Pansement médical selon l'une ou plusieurs des revendications précédentes, **caractérisé en ce que** la zone externe non adhésive présente, le long de la ligne de contour du pansement, une largeur de 0,5 à 5 mm, de préférence de 1,0 à 3,0 mm, de manière particulièrement préférée de 1,0 à 1,5 mm.

5. Pansement médical selon l'une ou plusieurs des revendications précédentes, **caractérisé en ce que** la zone externe non adhésive représente un bord appliqué sur la couche adhésive du pansement en une feuille ou un film en matériau synthétique.

6. Pansement médical selon l'une ou plusieurs des revendications précédentes, **caractérisé en ce que** le bord appliqué dépasse, au niveau de la surface, partiellement ou complètement des lignes de contour du reste du pansement.

7. Pansement médical selon l'une ou plusieurs des revendications précédentes, **caractérisé en ce que** la surface se trouvant côté peau de la zone externe présente un revêtement repoussant les auto-adhésifs, de préférence un siliconage ou un revêtement avec un polymère fluoré.

8. Pansement médical selon l'une ou plusieurs des revendications précédentes, **caractérisé en ce que** la zone externe non adhésive est réalisée avec une couleur ou une transparence différente par rapport au reste de la surface, de préférence par un dépôt par évaporation d'un métal sur une feuille participant à la structure à couches de la zone de bord.

9. Pansement médical selon l'une ou plusieurs des revendications précédentes, **caractérisé en ce qu'**au moins une substance active pharmaceutique est contenue.

10. Pansement médical selon l'une ou plusieurs des revendications précédentes, **caractérisé en ce que** la vitesse de libération de la substance active par la surface dans la zone externe est inférieure, d'au moins un facteur 10, à la vitesse de libération dans la zone interne contenant la substance active, de préférence d'au moins un facteur 100.

11. Pansement médical selon l'une ou plusieurs des revendications précédentes, **caractérisé en ce que** le pansement reste appliqué pendant 3 à 7 jours.

12. Pansement médical selon l'une ou plusieurs des revendications précédentes, **caractérisé en ce qu'**il contient des substances actives pour une thérapie hormonale de substitution ou pour la contraception transdermique.

13. Pansement médical selon l'une ou plusieurs des revendications précédentes, **caractérisé en ce que** la substance active est un gestagène et/ou un estrogène.

14. Pansement médical selon l'une ou plusieurs des revendications précédentes, **caractérisé en ce qu'**il présente une teneur en gestodène en combinaison avec un estrogène.

15. Procédé pour la réalisation d'un pansement médical selon la revendication 1, comprenant la réalisation d'un laminé pour la zone interne, constitué par une couche arrière, au moins une couche auto-adhésive contenant une substance active et de préférence une feuille de protection pouvant être retirée, ainsi que l'utilisation d'une feuille revêtue d'un côté de manière anti-adhésive pour la zone externe, **caractérisé en ce qu'**il comprend les étapes suivantes :
a) dans une étape, on réalise, dans la feuille prévue pour la zone de bord, des découpes correspondant aux contours de la zone interne ultérieure et les zones découpées sont rejetées,
b) dans une autre étape, on assemble la feuille découpée dans l'étape a) avec la feuille de protection du pansement à réaliser en les superposant, où, dans le cas d'une surface revêtue de manière anti-adhésive, cette surface anti-adhésive de la feuille découpée se plaçant sur la surface également anti-adhésive de la feuille de protection,
c) dans une autre étape, le laminé de la zone interne, constitué par au moins une couche formant la matrice auto-adhésive ainsi que la couche arrière, est enlevé de la couche de protection le cas échéant prévue au préalable dans le procédé de réalisation, collé avec le côté auto-adhésif sur l'assemblage formé par la feuille découpée de la zone de bord et la feuille de protection, le côté auto-adhésif du laminé était assemblé, à plat, avec la surface non revêtue de la feuille de la zone de bord et via les découpes dans celle-ci, également directement avec la surface revêtue de manière anti-adhésive de la feuille de protection,
d) dans une dernière étape, la découpe de la ligne de contour externe du pansement est réalisée à travers toutes les couches du laminé formé, cette ligne de contour externe s'étendant à la distance prévue pour la largeur de la zone de bord autour des découpes réalisées dans l'étape a) dans la feuille de la zone de bord.

16. Procédé pour la réalisation du pansement médical selon la revendication 8, comprenant les étapes de procédé selon la revendication 15, **caractérisé en ce que** le laminé, en s'écartant de l'étape d), n'est pas découpé à travers toutes les couches, mais qu'on découpe la ligne de contour du pansement à travers toutes les couches sauf la feuille de protection, le laminé en excès étant retiré de la feuille de protection et rejeté, avant qu'on découpe ou coupe dans une autre étape le contour de la feuille de protection dépassant le bord du pansement.
